# EUROPEAN PATENT SPECIFICATION

(11) **EP 1 016 699 B1**
(45) Date of publication and mention of the grant of the patent: **16.08.2006**
(21) Application number: 00104422.1
(22) Date of filing: 13.06.1994
(51) Int. Cl.: C09D 9/00, C11D 3/20, C11D 3/50, C23G 5/032, G03F 7/42

(54) **Use of a solvent composition comprising an oxyisobutyric acid ester as a cleaning agent**
Verwendung einer Lösungsmittel-Zusammensetzung enthaltend ein Oxyisobuttersäureester als Reinigungsmittel
Utilisation d'une composition de solvant contenant un ester d'acide oxyisobutyrique comme agent de nettoyage

(30) Priority: 15.06.1993 JP 16737493; 22.06.1993 JP 17360693; 17.09.1993 JP 25368893; 24.12.1993 JP 34605693; 11.03.1994 JP 10345794
(43) Date of publication of application: 05.07.2000
(62) Divisional of application: 94109036.7
(73) Proprietor: MITSUBISHI RAYON CO., LTD., Tokyo 108-8506 (JP)
(72) Inventor: Takayanagi, Yasuyuki, Nitto Chem. Ind. Co., Ltd., Yokohama-shi, Kanagawa (JP); Endou, Satoshi, Nitto Chem. Ind. Co., Ltd., Yokohama-shi, Kanagawa (JP); Sugama, Naoki, Nitto Chem. Ind. Co., Ltd., Yokohama-shi, Kanagawa (JP)
(74) Representative: HOFFMANN EITLE

(56) References cited:
- EP-A- 0 347 924
- WO-A-92/06169
- PATENT ABSTRACTS OF JAPAN vol. 016, no. 110 (C-0920), 18 March 1992 (1992-03-18) & JP 03 284651 A (SHOWA DENKO KK;OTHERS: 01), 16 December 1991 (1991-12-16)

## Description

### FIELD OF THE INVENTION

This invention relates to the use of a solvent composition, and more particularly to the use of a solvent composition as a cleaning agent.

### BACKGROUND OF THE INVENTION

Solvents have played an important roll in the development of the chemical industry. In particular, solvents are used in cleaning agents for removing oils, such as cutting oil, process oil, anti-corrosive oil, lubricating oil, grease and pitch, solder fluxes, inks, and liquid crystals.

Solvent-solubility of inks widely varies depending on their kind, for example, the kind of the base polymer or the hardening mechanism, such as ultraviolet-curing, heat-curing or hardening by evaporation. Therefore, an ink remover must have a strong dissolving power to be applicable to any kind of ink.

In the production of liquid crystal displays, it is necessary to form a high-density electrode pattern on a glass substrate so as to make a fine display. However, it is not easy to keep dense lines of an electrode pattern insulated from each other. Existence of even a trace amount of a contaminant on the substrate causes display defects due to insufficient insulation or burnout due to continuous galvanic corrosion. This ultimately results in destruction of the display function. In particular, when a liquid crystal is injected into a liquid crystal cell, the liquid crystal adheres to unnecessary parts of the cell through capillary action. The adhered unnecessary liquid crystal, if left as such, will fail to provide a clear display image, and also contaminants in the air are taken up by dissolution, which tends to cause insufficient insulation. Therefore, the adhered unnecessary liquid crystal must be removed with a cell cleaner, but it is very difficult to completely remove the liquid crystal which has entered narrow gaps.

For these uses, solvents mainly comprising halogen type solvents, such as Freon 113 (1,1,2-trichloro-1,2,2-trifluoroethane), methyl chloroform (1,1,1-trichloroethane), and trichloroethylene, have been in wide use. In particular, Freon 113 has been used extensively because of its nonflammability, low toxicity, excellent stability, and dissolving power selective for various kinds of contaminants with no corrosion of metals, plastics or elastomers. However, because Freon 113 and methyl chloroform rise up to the stratosphere and destroy the ozonosphere, which eventually leads to induction of cancer of skin, their use has been severely restricted. Use of trichloroethylene is also now been restricted because it is suspected as being carcinogenic.

Accordingly, intensive studies have been conducted in order to secure a cleaning agent which will take the place of Freons, showing a cleaning action equal to the Freon 113, without entertaining a fear of destruction of the ozonosphere. For example, a number of substitutes for Freons have been proposed to date, including a composition mainly comprising 1,2-difluoroethane (see JP-A-1-132694), a mixture of 1,1-dichloro-2,2,2-trifluoroethane and dimethoxymethane (see JP-A-2-178396, corresponding to US Patent 5,068,051), and a composition mainly comprising hexafluorobenzene (see JP-A-3-167298). Furthermore, EP 0 347 924 discloses a halogenated hydrocarbon solvent consisting essentially of a hydrogen-containing chlorofluoropropane having a difluoromethylene group represented by the formula:

CHₐCl_{b}F_{c}CF₂CHₓCl_{y}F_{z},

wherein a + b + c = 3, x + y + z = 3, a + x ≥ 1, b + y ≥ 1, and 0 ≤ a, b, c, x, y, z ≤ 3.

The hydrocarbon solvent may be used e.g. as a cleaning agent. It may contain further organic solvents, such as a hydrocarbon, an alcohol, a ketone, a chlorinated hydrocarbon, an ester, an aromatic compound or a surfactant in an amount of from 0 to 80% by weight. The ester may be *inter alia* ethyl 2-hydroxy-2-methylpropionate.

However, none of these solvent compositions offers a complete solution to the above-mentioned problems, i.e., they do not compare with Freon 113 in terms of performance. In addition, there is a movement to restrict use of these halogen type solvents.

On the other hand, cleaning agents for removal of fats and oils, i.e., degreasing agents, which are highly safe to humans and which do not cause environmental destruction have been proposed. For example, a composition mainly comprising a nonionic surface active agent and an alkyl lactate (see JP-A-4-68088), a composition mainly comprising a nonionic surface active agent and an adipic ester (see JP-A-4-59985), a composition mainly comprising a nonionic surface active agent and a polyalkylene glycol dialkyl ether (see JP-A-4-59984), a composition mainly comprising a nonionic surface active agent and N-methylpyrrolidone, etc. (see JP-A-4-68094), a composition mainly comprising a nonionic surface active agent and a glycerin acetate compound (see JP-A-4-68092), and a composition mainly comprising an alcohol and a fatty acid ester (see JP-A-4-68090) have been proposed. Although an alkyl lactate, N-methylpyrrolidone, etc. are highly safe solvents in terms of low toxicity, and do not cause environmental destruction or accumulate in the environment, they have insufficient dissolving power for fats and oils when used alone, as is obvious from the Comparative Examples given in the aforementioned patent publications. They essentially need a combined use of a detergent aid, such as a surface active agent, for application as a degreasing agent.

A solvent composition mainly comprising an alkyl lactate which is highly safe to humans and does not cause environmental destruction has been proposed as a cleaning agent for removing inks, i.e., an ink remover, as disclosed in JP-A-3-41170. While an alkyl lactate is a highly safe solvent in terms of low toxicity, and does not cause environmental destruction or accumulate in the,environment, it is still unsatisfactory as an ink remover due to insufficient dissolving power for high polymer-based inks.

As discussed above, it has been desired in the art to develop a solvent system which substitutes for Cellosolve acetate, Freon 113, methyl chloroform, etc., while exhibiting a high dissolving power for high polymers, fats and oils, solder fluxes, liquid crystals, agricultural chemicals, cosmetics, and various compounding additives, without giving rise to safety problems.

### SUMMARY OF THE INVENTION

An object of the present invention is to provide a solvent composition for use as a cleaning agent freed of the drawbacks of conventional solvents, such as Cellosolve acetate, Freon 113 and methyl chloroform, comprising a solvent system which is of low toxicity and harmless to humans, has high dissolving power, does not produce environment destructive substances, does not give off offensive odor, and has a relatively high boiling point indicative of safety and ease in handling.

As a result of extensive research into a solvent composition having the above-described favorable properties, the present inventors have found that the object of the present invention can be met by an oxyisobutyric acid ester selected from an alkyl α-alkoxyisobutyrate, alkyl β-alkoxyisobutyrate, and alkyl α-hydroxyisobutyrate. The present invention has been completed based on this finding.

The present invention provides the use of a solvent composition containing, as an active component, at least one oxyisobutyric acid ester selected from the group consisting of an alkyl α-alkoxyisobutyrate represented by formula (I): 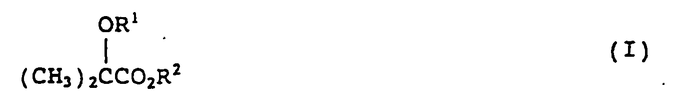 an alkyl β-alkoxyisobutyrate represented by formula (II): 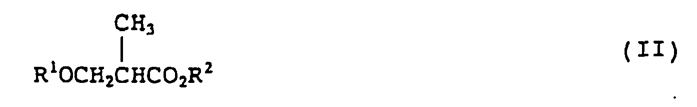 and an alkyl α-hydroxyisobutyrate represented by formula (III): 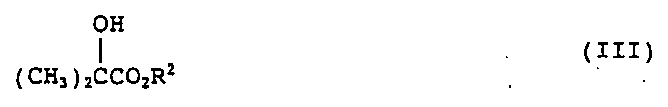 wherein R¹ and R² each represent an alkyl group having from 1 to 4 carbon atoms, in an amount of at least 5% by weight, as a cleaning agent.

### DETAILED DESCRIPTION OF THE INVENTION

The oxyisobutyric acid esters represented by formulae (I), (II) and (III), which can be used in the present invention, are available as disclosed, for example, in EP-A-429800.

The solvent composition of the present invention essentially contains an alkyl oxyisobutyrate. The alkyl oxyisobutyrates include alkyl α-alkoxyisobutyrates (I), such as methyl α-methoxyisobutyrate, ethyl α-methoxyisobutyrate, methyl α-ethoxyisobutyrate, and ethyl α-ethoxyisobutyrate; alkyl β-alkoxyisobutyrates (II), such as methyl β-methoxyisobutyrate, ethyl β-methoxyisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate, ethyl β-butoxyisobutyrate, and butyl β-butoxyisobutyrate; and alkyl α-hydroxyisobutyrates (III), such as methyl α-hydroxyisobutyrate, ethyl α-hydroxyisobutyrate, isopropyl α-hydroxyisobutyrate, and butyl α-hydroxyisobutyrate. From the standpoint of dissolving power and volatility, methyl α-methoxyisobutyrate, ethyl α-ethoxyisobutyrate, methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate, are preferred.

The solvent composition of the present invention is also useful as a degreasing agent, an ink remover, a flux remover, a liquid crystal cell cleaner or a resist stripper.

Alkyl oxyisobutyrates which are particularly useful as a degreasing agent include methyl α-methoxyisobutyrate, ethyl α-ethoxyisobutyrate, methyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate.

Alkyl oxyisobutyrates which are particularly useful as an ink remover include methyl α-methoxyisobutyrate, methyl β-methoxyisobutyrate, ethyl β-methoxyisobutyrate, methyl β-ethoxyisobutyrate, ethyl β-ethoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, and butyl α-hydroxyisobutyrate.

Alkyl oxyisobutyrates which are particularly useful as a flux remover include methyl α-methoxyisobutyrate, ethyl α-ethoxyisobutyrate, methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate.

Alkyl oxyisobutyrates which are particularly useful as a liquid crystal cell cleanser include methyl α-methoxyisobutyrate, methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, and butyl α-hydroxyisobutyrate.

Alkyl oxyisobutyrates which are particularly useful as a resist stripper include methyl α-methoxyisobutyrate, ethyl α-ethoxyisobutyrate, methyl β-methoxyisobutyrate, ethyl β-methoxyisobutyrate, methyl β-ethoxyisobutyrate, ethyl β-ethoxyisobutyrate, methyl β-isopropbxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate.

The alkyl α-alkoxyisobutyrates represented by formula (I), alkyl β-alkoxyisobutyrates represented by formula (II) and alkyl α-hydroxyisobutyrates represented by formula (III) may be used either individually or in combination of two or more thereof. While not limiting in combination, alkyl oxyisobutyrates represented by formula (I) or (II) is preferably used in combination with α-hydroxyisobutyrates represented by formula (III), in order to take advantage of dissolving power. The proportion of the alkyl oxyisobutyrates represented by formula (I) or (II)/α-hydroxyisobutyrates represented by formula (III) is preferably from 5/95 to 95/5 by weight, more preferably from 10/90 to 90/10 by weight, most preferably from 30/70 to 70/30 by weight.

The alkyl α-alkoxyisobutyrates, alkyl β-alkoxyisobutyrates, and alkyl α-hydroxyisobutyrates are compatible with other general organic solvents, such as alcohols, esters, ketones, amides, and aromatic hydrocarbons. They exhibit markedly excellent dissolving power for a wide range of high polymers, which include natural resins, such as cellulose resins, and synthetic resins, such as epoxy resins, acrylic resins, vinyl resins (e.g., vinyl acetate resins and vinyl chloride resins), alkyd resins, polyester resins, and novolak resins, as well as general hydrocarbon-based fats and oils. Accordingly, the oxyisobutyric esters of the present invention not only serve as a solvent by themselves, but they also exhibit excellent performance as a diluent or an auxiliary solvent for other organic solvents. Thus, they can be formulated into a solvent composition together with other organic solvents. The proportion of the oxyisobutyric ester in the solvent composition is not less than 5% by weight, and preferably not less than 10% by weight, in order to take full advantage of the safety and dissolving power of the oxyisobutyric ester.

The other solvents with which the oxyisobutyric esters of the present invention may be combined are not particularly limited and include water, alcohols, ethers, esters, ketones, amides, aliphatic hydrocarbons, and aromatic hydrocarbons. Suitable examples of the other solvents are water, methanol, ethanol, isopropyl alcohol, butanol, methyl isobutyl carbinol, heptanol, octanol, nonanol, 3-methylbutanol, propylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane. Preferred of them are water, methanol, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly preferred to provide a degreasing agent include isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly useful to provide an ink remover include water, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly useful to provide a flux remover include water, methanol, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, nitromethane, nitroethane, toluene, and xylene.

Solvents which are particularly useful to provide a liquid crystal cell cleaner include isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, nitromethane, nitroethane, toluene, and xylene.

Solvents which are particularly useful to provide a resist stripper include ethanol, isopropyl alcohol, butanol, 3-methylbutanol, 3-methoxybutanol, 3-methyl-3-methoxybutanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

These organic solvents and water may be used either individually or in combination of two or more thereof. A combined use of the organic solvent or water makes it possible to appropriately improve or modify the cleaning properties, safety, ease in handling, and the like of the solvent composition of the present invention.

Since the alkyl oxyisobutyrates of the present invention have a high boiling point and a relatively low rate of evaporation, they are useful as high-boiling solvents. When compounded into mixed solvent systems, they bring about improvements in performance properties and workability of coating compositions, adhesives, ink compositions, etc., such as spreadability and smoothness of a coating film and effects on fusion of resins.

The content of the alkyl oxyisobutyrate in the solvent composition is not less than about 5% by weight, and preferably not less than 10% by weight, depending on the use.

When used as a cleaning agent, the cleansing action of the solvent composition may be improved, if desired, by using a surface active agent, such as a nonionic surface active agent (e.g., polyalkyleneoxides and alkanolamides), a anionic surface active agent (e.g., alkyl(aryl)sulfonic acids and alkylphosphonic acids) or a cationic surface active agent (e.g., long chain amines and quaternary ammonium salts), an acidic compound, or a basic compound in combination. The typical example of the surface active agent includes polyoxyethylene dodecyl ether, sodium dodecylbenzenesulfonic acid and trimethylbenzylammonium chloride. The typical example of the acidic compound includes acetic acid, α-methoxyisobutyric acid, β-methoxyisobutyric acid, α-hydroxyisobutyric acid and methacrylic acid. The typical example of the basic compound includes triethylamine and triethanolamine. The surface active agent, acidic compound and basic compound is preferably used in an amount of from about 0.01 to 30% by weight, more preferably from 0.1 to 10% by weight, based on the total solvent composition.

When used as a resist stripper, the solvent composition may further contain a stripping accelerator, such as benzenesulfonic acid, toluenesulfonic acid, xylenesulfonic acid, phenolsulfonic acid, and alkylbenzenesulfonic acids, e.g., methyl-, propyl-, heptyl-, octyl-, decyl- or dodecylbenzenesulfonic acid. The stripping accelerator is usually used in an amount of from about 5 to 30% by weight, preferably 7 to 20% by weight, based on the total solvent composition. To improve stripping properties, the composition may furthermore contain a surface active agent, such as a nonionic surface active agent, anionic surface active agent or a cationic surface active agent, an acidic compound, or a basic compound.

The oxyisobutyric esters of the present invention have very high dissolving power for various organic compounds, including high polymers and naturally-occurring compounds. On the other hand, the alkyl oxyisobutyrates of the present invention do not have risk of explosion at an ordinal temperature, because their ignition point is 40°C or more. In addition, the acute toxicity (median lethal dose (LD₅₀); rat, oral) of the alkyl oxyisobutyrates is 2000mg/kg or more.

The photoresists to which the resist stripper of the present invention is applicable are not limited at all. That is, the resist stripper of the invention is useful for removal of any of positive or negative resists for optical alignment, resists for far ultraviolet light alignment, and resists for X-ray or electron beam alignment. Main materials known for these resists include novolak resins, cyclized rubbers, polysilicic acid, (meth)acrylic resins, (meth)acrylic acid copolymers, and polyhydroxystyrene. The resist stripper of the present invention is effective on any of these high polymers.

The present invention will now be illustrated in greater detail with reference to the Examples in view of the Comparative Examples, but the present invention should not be construed as being limited thereto. All of the mixing ratios of the solvents are given by weight unless otherwise indicated.

### EXAMPLE 1

The compatibility of the alkyl oxyisobutyrates of the present invention with other solvents was examined as follows.

Methyl α-methoxyisobutyrate, methyl β-methoxyisobutyrate, methyl α-hydroxyisobutyrate or a 30/70 mixture of methyl β-methoxyisobutyrate and methyl α-hydroxyisobutyrate, and the solvents shown in Table 1 below were mixed in a mixing ratio of 1/1 by volume in an Erlenmeyer flask, and the mixture was allowed to stand still at room temperature. The degree of compatibility was visually observed and rated as follows. The results obtained are shown in Table 1.
- A: Completely uniformly mixed.
- B: White turbidity observed.
- C: Separated into two phases.

### COMPARATIVE EXAMPLE 1

The compatibility of Cellosolve acetate (ethylene glycol monoethyl ether acetate; hereinafter abbreviated as "ECA") with the solvent shown in Table 1 was examined in the same manner as in Example 1. The results obtained are shown in Table 1 below.

**TABLE 1**

| | Example 1 | | | | Compar. Example 1 |
|---|---|---|---|---|---|
| Solvent | α-MBM¹⁾ | β-MBM²⁾ | α-HBM³⁾ | β-MBM/ α-HBM⁴⁾ | ECA |
| Water | C | C | A | B | C |
| Methanol | A | A | A | A | A |
| Ethanol | A | A | A | A | A |
| Isopropyl alcohol | A | A | A | A | A |
| Ethyl acetate | A | A | A | A | A |
| Acetone | A | A | A | A | A |
| dimethylformamide | A | A | A | A | A |
| Acetonitrile | A | A | A | A | A |
| Benzene | A | A | A | A | A |
| Toluene | A | A | A | A | A |
| Xylene | A | A | A | A | A |
| n-Hexane | A | A | A | A | A |
| Cyclohexane | A | A | A | A | A |
| Cyclohexanone | A | A | A | A | A |
| Cyclohexanol | A | A | A | A | A |

| | | | | | |
|---|---|---|---|---|---|
| Note: 1): Methyl α-methoxyisobutyrate | | | | | |
| 2): Methyl β-methoxyisobutyrate | | | | | |
| 3): Methyl α-hydroxyisobutyrate | | | | | |
| 4): β-MBM/α-HBM = 30/70 | | | | | |

### EXAMPLE 2

The rate of evaporation of the alkyl oxyisobutyrates of the present invention was measured as follows.

The solvents shown in Table 2 below weighing 0.75 g, were uniformly spread on a disc of No. 4 filter paper having a diameter of 95 mm. The coated filter paper was allowed to stand on a testing stand at 25°C with a covering over it. The sample was weighed at given time intervals to determine the evaporation loss, from which the rate of evaporation was calculated. The results obtained, expressed relatively taking the rate of evaporation of butyl acetate as a standard (100), are shown in Table 2 below.

**TABLE 2**

| Solvent | Relative Rate of Evaporation |
|---|---|
| Butyl acetate | 100 (standard) |
| Methyl α-hydroxyisobutyrate | 56 |
| Ethyl α-Hydroxyisobutyrate | 26 |
| Methyl α-methoxyisobutyrate | 64 |
| Methyl β-methoxyisobutyrate | 44 |
| Ethyl β-ethoxyisobutyrate | 16 |

### COMPARATIVE EXAMPLE 2

The rate of evaporation of the conventional solvents shown in Table 3 below was determined in the same manner as in Example 2. The results obtained are shown in Table 3 below.

**TABLE 3**

| Solvent | Relative Rate of Evaporation |
|---|---|
| Butyl acetate | 100 (standard) |
| ECA | 22 |
| Ethyl lactate | 23 |
| Methyl β-methoxypropionate | 35 |

### EXAMPLE 3

The dissolving power of methyl β-methoxyisobutyrate for various resins was evaluated as follows.

In a 100 mℓ-volume Erlenmeyer flask was put 2.0 g of the resins shown in Table 4 below, and 20 mℓ of methyl β-methoxyisobutyrate was added thereto. The mixture was kept at 25°C while stirring, and the time required for the resin to dissolve was measured. The results obtained are shown in Table 4 below.

### COMPARATIVE EXAMPLE 3

The dissolving power of ECA was evaluated in the same manner as in Example 3. The results are shown in Table 4 below.

**TABLE 4**

| | Time for Dissolution (min) | |
|---|---|---|
| Resin | Example 3 | Compar. Example 3 |
| Polyester (ER-1002, a product of Mitsubishi Rayon Co., Ltd.) | 110 | 150 |
| Polystyrene (QPX2B, a product of Denki Kagaku Kogyo K.K. | 46 | 86 |
| Methyl methacrylate-styrene copolymer (MS-300, a product of Nippon Steel Chemical Co., Ltd.) | 82 | 125 |
| Acrylonitrile-styrene copolymer (AS-30, a product of Asahi Chemical Industry Co., Ltd.) | 95 | 145 |
| Acrylic resin (Acrydic A-405, a product of Dainippon Ink and Chemicals, Inc.) | 30 | 45 |
| Phenoxy resin (PKH-H, a product of Union Carbide Corp.) | 110 | 150 |
| Polysulfone (P1800NT11, a product of Amoco Co.) | 160 | 260 |

### EXAMPLE 4

The dissolving power of the alkyl oxyisobutyrates shown in Table 5 below for an epoxy resin was evaluated.

In a 100 mℓ-volume Erlenmeyer flask was put 2.0 g of an epoxy resin (Epikote 1007, a product of Yuka Shell Epoxy Co., Ltd.), and 20 mℓ of the alkyl oxyisobutyrate was added thereto. The mixture was kept at 25°C while stirring, and the time required for the resin to dissolve was measured. The results obtained are shown in Table 5 below.

**TABLE 5**

| Solvent | Time of Dissolution |
|---|---|
| | (min) |
| Methyl α-methoxyisobutyrate | 22 |
| Methyl β-methoxyisobutyrate | 25 |
| Ethyl β-ethoxyisobutyrate | 28 |
| Methyl α-methoxyisobutyrate/ methyl α-hydroxyisobutyrate (50/50) | 29 |
| Methyl α-hydroxyisobutyrate | 38 |

### COMPARATIVE EXAMPLE 4

The epoxy resin-dissolving power of ECA, ethyl lactate or methyl β-methoxypropionate was evaluated in the same manner as in Example 4. The results are shown in Table 6 below.

**TABLE 6**

| Solvent | Time of Dissolution |
|---|---|
| | (min) |
| ECA | 45 |
| Ethyl lactate | 65 |
| Methyl β-methoxypropionate | 42 |

As can be seen from Table 1, the oxyisobutyric esters of the present invention have compatibility equal or superior to the conventional general-purpose solvents, such as ECA, and thus can be used as a mixture with a broad range of other solvents.

As can be seen from Tables 2 and 3, the a rate of evaporation can arbitrarily be selected by a proper choice of the ester group. The rate of evaporation of the oxyisobutyric esters of the present invention is about 2 to 3 times as high as that of ECA or ethyl lactate, which have been widely used, proving that the oxyisobutyric esters provide a solvent system having good volatility.

As can be seen from Tables 4, 5 and 6, the oxyisobutyric esters of the present invention need a shorter time for dissolving various resins, often used in coating compositions and adhesives, than needed by the conventional general-purpose solvents. This reveals their excellent resin-dissolving power.

### EXAMPLE 5

A 50 mm long, 20 mm wide, and 2 mm thick stainless steel plate with its surface polished was uniformly coated with commercially available fats and oils, i.e., cutting oil (Daphne-Mag plus LA15, a product of Idemitsu Kosan Co., Ltd.), press oil (Unipress DP120, a product of Nippon Oil Co., Ltd.) or grease (Albania Grease 1, a product of Showa Shell Sekiyu K.K.). One hour later, the coated plate was put in a washing bottle containing the degreasing agent shown in Table 7 below, and the bottle and content was shaken at 40°C for 5 minutes. The stainless steel plate was taken out and dried in hot air. The oil or grease remaining on the plate was observed with the naked eye and under a metallurgical microscope, and the degreasing power was evaluated according to the following rating system.
- A: No residual oil or grease was observed with the naked eye or under a metallurgical microscope.
- B: Residual oil or grease was not observed with the naked eye, but observed under a metallurgical microscope.
- C: Residual oil or grease was observed with the naked eye.

The results obtained are shown in Table 7 below.

**TABLE 7**

| | Degreasing Power | | |
|---|---|---|---|
| Degreasing Agent | Cutting Oil | Press Oil | Grease |
| Methyl α-methoxyisobutyrate | A | A | A |
| Methyl β-methoxyisobutyrate | A | A | A |
| Ethyl α-methoxyisobutyrate/ isopropyl alcohol (50:50) | A | A | A |
| Methyl β-methoxyisobutyrate/methyl α-hydroxyisobutyrate (30:70) | A | A | A |
| Ethyl β-ethoxyisobutyrate/ methyl isobutyl ketone (80:20) | A | A | A |
| Methyl β-ethoxyisobutyrate/ dimethylformamide (50:50) | A | A | A |
| Methyl β-methoxyisobutyrate/N-methylpyrrolidone/water (50:40:10) | A | A | A |
| Methyl α-methoxyisobutyrate/methyl β-methoxyisobutyrate (30:70) | A | A | A |
| Methyl α-hydroxyisobutyrate | A | A | B |
| Ethyl α-hydroxyisobutyrate/ethyl lactate/polyoxyethylene dodecyl ether (10:80:10) | A | A | A |
| Methyl β-methoxyisobutyrate/butyl acetate/octanol (50:20:30) | A | A | A |
| Methyl β-methoxyisobutyrate/ isophorone (70:30) | A | A | A |
| Methyl α-hydroxyisobutyrate/3-methoxybutyl acetate (50:50) | A | A | A |
| Methyl β-methoxyisobutyrate/amyl acetate/3-methoxybutanol (30:50:20) | A | A | A |
| Methyl α-hydroxyisobutyrate/N-methylpyrrolidone/water (30:50:20) | A | B | A |
| Methyl β-methoxyisobutyrate/2-ethyl-hexanol/dibenzyl ether (50:30:20) | A | A | A |
| Methyl β-methoxyisobutyrate/2-ethylhexyl acetate/benzyl alcohol (50:30:20) | A | A | A |
| Methyl α-methoxyisobutyrate/3,5,5-trimethyl-1-hexanol/benzyl acetate (40:30:30) | A | A | A |
| Methyl α-hydroxyisobutyrate/ cyclohexanol/cyclohexyl acetate (60:20:20) | A | A | A |

### COMPARATIVE EXAMPLE 5

The conventional degreasing agents shown in Table 8 below were tested in the same manner as in Example 5. The results obtained are shown in Table 8 below.

**TABLE 8**

| | Degreasing Power | | |
|---|---|---|---|
| Degreasing Agent | Cutting Oil | Press Oil | Grease |
| 1,1,1-Trichloroethane | A | B | A |
| Freon 113 | A | C | B |
| Ethyl lactate | C | C | C |
| N-Methylpyrrolidone | B | C | C |

### EXAMPLE 6

Various commercially available inks were uniformly spread on a 50 mm long, 20 mm wide, and 2 mm thick aluminum plate with a polished surface by means of a bar coder. One hour later, the coated aluminum plate was put in a washing bottle containing the ink removers shown in Table 9 below, and the bottle and content was shaken at room temperature for 30 seconds. The plate was taken out and dried. The ink remaining on the plate was observed with the naked eye and under a metallurgical microscope, and the ink removing power was evaluated according to the following rating system.
- A: No residual ink was observed with the naked eye or under a metallurgical microscope.
- B: Residual ink was not observed with the naked eye, but observed under a metallurgical microscope.
- C: Residual ink was observed with the naked eye.

The results obtained are shown in Table 9 below.

**TABLE 9**

| | Ink Removing Power | | | |
|---|---|---|---|---|
| Ink Remover | 1* | 2* | 3* | 4* |
| Methyl α-methoxyisobutyrate | A | A | A | A |
| Methyl β-methoxyisobutyrate | A | A | A | A |
| Ethyl α-methoxyisobutyrate/ isopropyl alcohol (50:50) | A | A | A | A |
| Methyl β-methoxyisobutyrate/methyl α-hydroxyisobutyrate (30:70) | A | A | A | B |
| Ethyl β-ethoxyisobutyrate/methyl isobutyl ketone (80:20) | A | A | A | A |
| Methyl β-ethoxyisobutyrate/ dimethylformamide (50:50) | A | A | A | A |
| Methyl β-methoxyisobutyrate/N-methylpyrrolidone/water (50:40:10) | A | A | A | A |
| Methyl α-methoxyisobutyrate/methyl β-methoxyisobutyrate (30:70) | A | A | A | A |
| Methyl α-hydroxyisobutyrate | A | A | B | B |
| Ethyl α-hydroxyisobutyrate/ethyl lactate/dodecylbenzenesulfonic acid (10:80:10) | A | A | A | A |
| Methyl β-methoxyisobutyrate/butyl acetate/octanol (50:20:30) | A | A | A | A |
| Methyl β-methoxyisobutyrate/ isophorone (70:30) | A | A | A | A |
| Methyl α-hydroxyisobutyrate/3-methoxybutyl acetate (50:50) | A | A | A | B |
| Methyl β-methoxyisobutyrate/amyl acetate/3-methoxybutanol (30:50:20) | A | A | A | A |
| Methyl α-hydroxyisobutyrate/N-methylpyrrolidone/water/acetic acid (30:50:15:5) | A | A | A | A |
| Methyl β-methoxyisobutyrate/2-ethylhexanol/dibenzyl ether (50:30:20) | A | A | A | A |
| Methyl β-methoxyisobutyrate/2-ethylhexyl acetate/benzyl alcohol (50:30:20) | A | A | A | A |
| Methyl α-methoxyisobutyrate/3,5,5-trimethyl-1-hexanol/benzyl acetate (40:30:30) | A | A | A | A |
| Methyl α-hydroxyisobutyrate/ cyclohexanol/cyclohexyl acetate (60:20:20) | A | A | A | A |

| | | | | |
|---|---|---|---|---|
| Note: 1*: Cellulose resin-based green ink for printing letters on instrument boards | | | | |
| 2*: Hot-setting green solder resist ink | | | | |
| 3*: UV-curing white marking ink | | | | |
| 4*: Vinyl chloride-acrylate copolymer-based white screen printing ink | | | | |

### COMPARATIVE EXAMPLE 6

The conventional ink removers shown in Table 10 below were tested in the same manner as in Example 6. The results obtained are shown in Table 10 below.

**TABLE 10**

| | Ink Removing Power | | | |
|---|---|---|---|---|
| Ink Remover | 1* | 2* | 3* | 4* |
| 1,1,1-Trichloroethane | A | B | A | C |
| Ethyl lactate | A | C | B | C |
| 1,1,1-Trichloroethane/ cyclohexanone (80:20) | A | A | A | B |
| Methyl β-methoxypropionate | B | B | A | B |

### EXAMPLE 7

A commercially available flux (Solderite flux B-111R, a product of Tamura Corporation) was uniformly applied to the entire surface of a base of a printed circuit board (a copper-clad laminate), preliminarily dried at 100°C, and baked at 220°C.

The coated base was cleaned by immersing in the flux removers shown in Table 11 below while shaking at room temperature for 5 minutes. The base was taken out, washed with water, and dried. The residual flux on the base was observed with the naked eye and under a metallurgical microscope, and rated as follows.
- A: No residual flux was observed with the naked eye or under a metallurgical microscope.
- B: Residual flux was not observed with the naked eye, but observed under a metallurgical microscope.
- C: Residual flux was observed with the naked eye.

The results obtained are shown in Table 11 below.

**TABLE 11**

| Flux Remover | Flux Removing Power |
|---|---|
| Methyl α-methoxyisobutyrate | A |
| Methyl β-methoxyisobutyrate | A |
| Methyl α-methoxyisobutyrate/ethanol (50:50) | A |
| Methyl β-methoxyisobutyrate/methyl α-hydroxyisobutyrate (30:70) | A |
| Ethyl β-ethoxyisobutyrate/acetone (50:50) | A |
| Methyl β-ethoxyisobutyrate/dimethyl-formamide (50:50) | A |
| Methyl β-methoxyisobutyrate/N-methyl-pyrrolidone/water (40:50:10) | A |
| Methyl α-methoxyisobutyrate/methyl β-methoxyisobutyrate (30:70) | A |
| Methyl α-methoxyisobutyrate/methyl α-hydroxyisobutyrate (7:93) | A |
| Methyl β-methoxyisobutyrate/methyl lactate (30:70) | A |
| Methyl β-methoxyisobutyrate/butyl acetate/octanol (50:20:30) | A |
| Methyl β-methoxyisobutyrate/ isophorone (70:30) | A |
| Methyl α-methoxyisobutyrate/ 3-methoxybutyl acetate (50:50) | A |
| Methyl β-methoxyisobutyrate/amyl acetate/3-methoxybutanol (30:50:20) | A |
| Methyl β-methoxyisobutyrate/N-methylpyrrolidone/water/acetic acid (30:50:15:5) | A |
| Methyl β-methoxyisobutyrate/2-ethylhexanol/dibenzyl alcohol (50:30:20) | A |
| Methyl β-methoxyisobutyrate/2-ethyl-hexyl acetate/benzyl alcohol (50:30:20) | A |
| Methyl α-methoxyisobutyrate/3,5,5-trimethyl-1-hexanol/benzyl acetate (40:30:30) | A |
| Methyl α-hydroxyisobutyrate/cyclo-hexanol/cyclohexyl acetate (60:20:20) | A |

### COMPARATIVE EXAMPLE 7

The conventional flux removers shown in Table 12 below were tested in the same manner as in Example 7. The results obtained are shown in Table 12 below.

**TABLE 12**

| Flux Remover | Flux Removing Power |
|---|---|
| Freon 113 | C |
| Methyl lactate | B |

### EXAMPLE 8

A commercially available flux (Tamura F-A1-4, a product of Tamura Corporation) was uniformly applied to the entire surface of a base of a printed circuit board (a copper-clad laminate), preliminarily dried at 100°C, and baked at 220°C.

The coated base was cleaned by immersing in the flux removers shown in Table 13 below while shaking at 40°C for 5 minutes. The base was taken out, washed with water, and dried. The residual flux on the base was observed with the naked eye and under a metallurgical microscope, and rated according to the same standard as in Example 7.

The results obtained are shown in Table 13 below.

**TABLE 13**

| Flux Remover | Flux Removing Power |
|---|---|
| Methyl α-hydroxyisobutyrate | A |
| Ethyl α-hydroxyisobutyrate | A |
| Methyl α-hydroxyisobutyrate/dimethyl-formamide (70:30) | A |
| Butyl α-hydroxyisobutyrate/nitro-methane (70:30) | A |
| Methyl α-hydroxyisobutyrate/ethanol (90:10) | A |
| Isopropyl α-hydroxyisobutyrate | A |
| Methyl α-hydroxyisobutyrate/water (90:10) | A |
| Methyl α-hydroxyisobutyrate/methyl lactate (50:50) | A |
| Methyl α-hydroxyisobutyrate/ methyl β-methoxyisobutyrate (70:30) | A |
| Methyl α-hydroxyisobutyrate/butyl acetate/octanol (40:30:30) | A |
| Ethyl α-hydroxyisobutyrate/3-methyl-3-methoxybutanol/cyclohexanone (10:40:50) | A |
| Methyl α-hydroxyisobutyrate/2-ethyl-hexanol/dibenzyl ether (50:30:20) | A |
| Methyl α-hydroxyisobutyrate/2-ethyl-hexyl acetate/benzyl alcohol (50:30:20) | A |
| Methyl α-hydroxyisobutyrate/cyclo-hexanol/cyclohexyl acetate (60:20:20) | A |

### COMPARATIVE EXAMPLE 8

The conventional flux removers shown in Table 14 below were tested in the same manner as in Example 8. The results obtained are shown in Table 14 below.

**TABLE 14**

| Flux Remover | Flux Removing Power |
|---|---|
| Freon 113 | C |
| Methyl lactate | B |

### EXAMPLE 9

A liquid crystal cell assembled from a pair of glass plates (60 x 30 x 1 mm), with the surface having been cleaned, and spacers to have a cell gap of 10 µm was filled with a commercially available liquid crystal (ZLI-1565, a product of Merck Japan Co., Ltd.) and sealed.

The liquid crystal cell was washed by soaking in 100 mℓ of the cell cleaners shown in Table 15 below at room temperature for 10 minutes. The cell was taken out and dried in hot air. The liquid crystal remaining on the glass plates and the periphery of the cell was observed with the naked eye and a polarizing microscope, and rated as follows.
- A: No residual liquid crystal was observed with the naked eye or under a polarizing microscope.
- B: Residual liquid crystal was not observed with the naked eye, but observed under a polarizing microscope.
- C: Residual liquid crystal was observed with the naked eye.

The results obtained are shown in Table 15 below.

**TABLE 15**

| Liquid Crystal Cleaner | Liquid Crystal Removing Power |
|---|---|
| Methyl α-methoxyisobutyrate | A |
| Methyl β-methoxyisobutyrate | A |
| Ethyl α-methoxyisobutyrate/isopropyl alcohol (50:50) | A |
| Methyl β-methoxyisobutyrate/methyl α-hydroxyisobutyrate (30:70) | A |
| Ethyl β-ethoxyisobutyrate/methyl isobutyl ketone (80:20) | A |
| Methyl β-methoxyisobutyrate/ dimethylformamide (50:50) | A |
| Methyl β-methoxyisobutyrate/ N-methylpyrrolidone (50:50) | A |
| Methyl α-methoxyisobutyrate/methyl β-methoxyisobutyrate (30:70) | A |
| Methyl α-hydroxyisobutyrate | B |
| Methyl β-methoxyisobutyrate/ethyl lactate/dodecylbenzenesulfonic acid (50:40:10) | A |
| Methyl β-methoxyisobutyrate/butyl acetate/octanol (50:20:30) | A |
| Methyl β-methoxyisobutyrate/ isophorone (70:30) | A |
| Methyl α-hydroxyisobutyrate/ 3-methoxybutyl acetate (50:50) | A |
| Methyl β-methoxyisobutyrate/amyl acetate/3-methoxybutanol (30:50:20) | A |
| Methyl α-hydroxyisobutyrate/N-methylpyrrolidone/water/polyoxy-ethylene dodecyl ether (40:40:15:5) | A |
| Methyl β-methoxyisobutyrate/2-ethylhexanol/dibenzyl ether (50:30:20) | A |
| Methyl β-methoxyisobutyrate/2-ethyl-hexyl acetate/benzyl alcohol (50:30:20) | A |
| Methyl α-methoxyisobutyrate/3,5,5-trimethyl-1-hexanol/benzyl acetate (40:30:30) | A |
| Methyl α-hydroxyisobutyrate/cyclo-hexanol/cyclohexyl acetate (60:20:20) | A |

### COMPARATIVE EXAMPLE 9

The conventional cell cleaners shown in Table 16 below were tested in the same manner as in Example 9. The results obtained are shown in Table 16 below.

**TABLE 16**

| Liquid Crystal Cleaner | Liquid Crystal Removing Power |
|---|---|
| 1,1,1-Trichloroethane | B |
| Polyoxyethylene butyl ether/ ethanol/water (30:35:35) | C |
| Ethyl lactate | C |
| Methyl β-methoxypropionate | B |

### EXAMPLE 10

A silicon wafer was uniformly coated with a commercially available positive photoresist for fine processing (OFPR-800, a product of Tokyo Ohka Kogyo Co., Ltd.) with a spinner (3000 rpm), pre-baked at 90°C for 30 minutes, and exposed to ultraviolet light to form a pattern. The resist was developed with an alkali developer (NMD-W, a product of Tokyo Ohka Kogyo Co., Ltd.) at 25°C for 1 minute, rinsed with pure water for 30 minutes, and post baked at 130°C for 30 minutes. Then, the resist was removed by immersing in the resist strippers shown in Table 17 below for 5 minutes while shaking, washed with water, and dried. The residual resist film was observed with the naked eye and under a metallurgical microscope, and rated as follows.
- A: No residual resist was observed with the naked eye or under a metallurgical microscope.
- B: Residual resist was not observed with the naked eye, but observed under a metallurgical microscope.
- C: Residual resist was observed with the naked eye.

The results obtained are shown in Table 17 below.

**TABLE 17**

| Resist Stripper | Resist Removing Power |
|---|---|
| Methyl α-methoxyisobutyrate | A |
| Methyl β-methoxyisobutyrate | A |
| Ethyl α-methoxyisobutyrate/ethanol/ dodecylbenzenesulfonic acid (50:40:10) | A |
| Methyl β-methoxyisobutyrate/methyl α-hydroxyisobutyrate/dodecylbenzenesulfonic acid (15:70:15) | A |
| Ethyl β-ethoxyisobutyrate/acetone (80:20) | A |
| Methyl β-ethoxyisobutyrate/dimethyl-formamide (50:50) | A |
| Methyl β-methoxyisobutyrate/N-methyl-pyrrolidone/water (50:40:10) | A |
| Methyl α-methoxyisobutyrate/methyl β-methoxyisobutyrate (30:70) | A |
| Methyl α-hydroxyisobutyrate | A |
| Ethyl α-hydroxyisobutyrate/ethyl lactate/dodecylbenzenesulfonic acid (10:70:20) | A |

### COMPARATIVE EXAMPLE 10

The conventional resist strippers shown in Table 18 below were tested in the same manner as in Example 10. The results obtained are shown in Table 18 below.

**TABLE 18**

| Resist Stripper | Resist Removing Power |
|---|---|
| ECA | C |
| Ethyl lactate | C |
| Ethyl lactate/dodecylbenzene-sulfonic acid (80:20) | B |
| Methyl β-methoxypropionate | B |
| Methyl β-methoxypropionate/ dodecylbenzenesulfonic acid (80:20) | A |

As described above, the solvent composition according to the present invention has excellent dissolving power, gives off no offensive smell, gives rise to no environmental problem, and is safe. Additionally, the solvent composition of the present invention has the following advantages:
1) It has an extremely high dissolving power for natural and synthetic high polymers;
2) It is freely miscible with many organic solvents;
3) It is biodegradable, non-accumulating in nature;
4) It has low toxicity, no teratogenicity, and high safety;
5) It has a relatively high boiling point and ignition point, so as to secure improved handling properties and safety; and
6) It is non-corrosive for a substrate.

The degreasing agent comprising the solvent composition of the present invention has high cleaning power. Since the degreasing agent has an extremely high dissolving power for various fats and oils as well as the above-mentioned advantages, it exhibits excellent ability in removal of fats, oils, and grease. Being water-soluble, the degreasing agent can be used in an aqueous system.

The ink remover comprising the solvent composition of the present invention has extremely high dissolving power for various types of inks, as well as the above-mentioned advantages, and therefore exhibits excellent ability in ink removal.

The flux remover comprising the solvent composition of the present invention exhibits an extremely high dissolving power for fluxes, as well as the above-mentioned advantages (1) to (6), and therefore has excellent ability in flux removal.

The liquid crystal cell cleaner comprising the solvent composition of the present invention has an extremely high dissolving power for various liquid crystals, as well as the above-mentioned advantages (1) to (6), and therefore exhibits excellent ability in removal of liquid crystals, and particularly contaminants from liquid crystal cells.

The resist stripper comprising the solvent composition of the present invention has an extremely high dissolving power for a photoresist, in addition to the above-mentioned effects (1) to (6), and exhibits excellent performance in removal of a resist.

## Claims

1. Use of a solvent composition comprising, as an active compound, at least one oxyisobutyric acid ester in an amount of at least 5 % by weight, wherein said oxyisobutyric acid ester is selected from the group consisting of an alkyl α-alkoxyisobutyrate represented by formula (I): 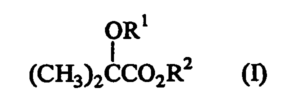 an alkyl β-alkoxyisobutyrate represented by formula (II): 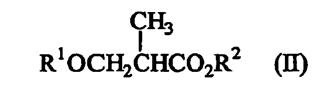 and an alkyl α-hydroxyisobutyrate represented by formula (III) : 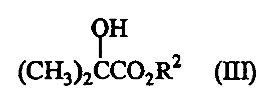 wherein R¹ and R² each represent an alkyl group having from 1 to 4 carbon atoms, provided that the composition does not contain halogen type solvents, as a cleaning agent.

2. Use according to claim 1 wherein the cleaning agent is a degreasing agent.

3. Use according to claim 1 wherein the cleaning agent is an ink remover.

4. Use according to claim 1 wherein the cleaning agent is a flux remover.

5. Use according to claim 1 wherein the cleaning agent is a liquid crystal cleaner.

6. Use according to claim 1 wherein the cleaning agent is a resist stripper.

7. Use as claimed in any of claims 1 to 6, wherein said solvent composition further comprises at least one compound selected from the group consisting of water, methanol, ethanol, isopropyl alcohol, butanol, methyl isobutyl carbinol, heptanol, octanol, nonanol, 3-methylbutanol, propylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane.

8. Use as claimed in any of claims 1 to 7, wherein said oxyisobutyric acid ester is selected from the group consisting of methyl α-methoxyisobutyrate, ethyl α-methoxyisobutyrate, methyl α-ethoxyisobutyrate, ethyl α-ethoxyisobutyrate, methyl β-methoxyisobutyrate, ethyl β-methoxyisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate, ethyl β-butoxyisobutyrate, butyl β-butoxyisobutyrate, methyl α-hydroxyisobutyrate, ethyl α-hydroxyisobutyrate, isopropyl α-hydroxyisobutyrate, and butyl α-hydroxyisobutyrate.

## Patentansprüche

1. Verwendung einer Lösungsmittelzusammensetzung, umfassend als Wirkstoff mindestens einen Oxyisobuttersäureester in einer Menge von mindestens 5 Gew.%, worin der Oxyisobuttersäureester ausgewählt ist aus der Gruppe bestehend aus einem Alkyl-α-alkoxyisobutyrat, dargestellt durch Formel (I): 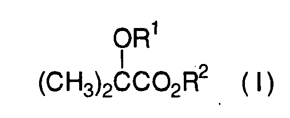 einem Alkyl-β-alkoxyisobutyrat, dargestellt durch Formel (II): 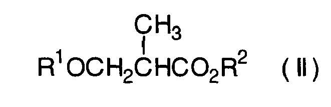 und einem Alkyl-α-hydroxyisobutyrat, dargestellt durch Formel (III): 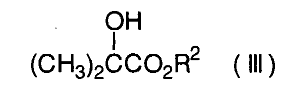 worin R¹ und R² jeweils eine Alkylgruppe mit 1 bis 4 Kohlenstoffatomen darstellen, vorausgesetzt, dass die Zusammensetzung keine Lösungsmittel vom Halogentyp enthält, als Reinigungsmittel.

2. Verwendung gemäss Anspruch 1, worin das Reinigungsmittel ein Entfettungsmittel ist.

3. Verwendung gemäss Anspruch 1, worin das Reinigungsmittel ein Tintenentferner ist.

4. Verwendung gemäss Anspruch 1, worin das Reinigungsmittel ein Flussmittelentferner ist.

5. Verwendung gemäss Anspruch 1, worin das Reinigungsmittel ein Flüssigkristallreiniger ist.

6. Verwendung gemäss Anspruch 1, worin das Reinigungsmittel ein Lackablöser ist.

7. Verwendung gemäss einem der Ansprüche 1 bis 6, worin die Lösungsmittelzusammensetzung ferner mindestens eine Verbindung umfasst, ausgewählt aus der Gruppe bestehend aus Wasser, Methanol, Ethanol, Isopropylalkohol, Butanol, Methylisobutylcarbinol, Heptanol, Octanol, Nonanol, 3-Methylbutanol, Propylenglykol, 3-Methoxybutanol, 3-Methyl-3-methoxybutanol, 3,5,5-Trimethyl-1-hexanol, 2-Ethyl-1-hexanol, Cyclohexanol, Benzylalkohol, Aceton, Methylethylketon, Methylisobutylketon, Cyclohexanon, Isophoron, Pyrrolidon, N-Methylpyrrolidon, Dimethylformamid, Dimethylacetamid, Dimethylsulfoxid, Sulfolan, Methylacetat, Ethylacetat, Butylacetat, Amylacetat, 3-Methoxybutylacetat, Butylacetat, Amylacetat, 3-Methoxybutylacetat, 3-Methyl-3-methoxybutylacetat, Methyllactat, Ethyllactat, Butyllactat, Methyl-3-methoxypropionat, Ethyl-3-ethoxypropionat, 2-Ethylhexylacetat, Cyclohexylacetat, Benzylacetat, Dibenzylether, Nitromethan, Nitroethan, Acetonitril, γ-Butyrolacton, Propylenglykolmonomethyletheracetat, Toluol, Xylol, Hexan und Cyclohexan.

8. Verwendung gemäss einem der Ansprüche 1 bis 7, worin der Oxyisobuttersäureester ausgewählt ist aus der Gruppe bestehend aus Methyl-α-methoxyisobutyrat, Ethyl-α-ethoxyisobutyrat, Methyl-β-methoxyisobutyrat, Ethyl-β-methoxyisobutyrat, Methyl-β-ethoxyisobutyrat, Ethyl-β-ethoxyisobutyrat, Methyl-β-isopropoxyisobutyrat, Ethyl-β-isopropoxyisobutyrat, Butyl-β-isopropoxyisobutyrat, Methyl-β-butoxyisobutyrat, Ethyl-β-butoxyisobutyrat, Butyl-β-butoxyisobutyrat, Methyl-α-hydroxyisobutyrat, Ethyl-α-hydroxyisobutyrat, Isopropyl-α-hydroxyisobutyrat und Butyl-α-hydroxyisobutyrat.

## Revendications

1. Utilisation d'une composition de solvant comprenant comme composé actif au moins un ester d'acide oxyisobutyrique dans une quantité d'au moins 5 % en poids, dans laquelle ledit ester d'acide oxyisobutyrique est choisi parmi un α-alcoxyisobutyrate d'alkyle représenté par la formule (I) : 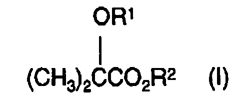 un β-alcoxyisobutyrate d'alkyle représenté par la formule (II) : 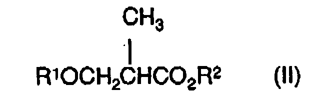 et un α-hydroxyisobutyrate d'alkyle représenté par la formule (III) : 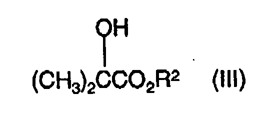 dans lesquelles R¹ et R² représentent chacun un groupe alkyle ayant de 1 à 4 atomes de carbone à condition que la composition ne contienne pas de solvants de type halogène, comme agent nettoyant.

2. Utilisation selon la revendication 1, dans laquelle l'agent nettoyant est un agent dégraissant.

3. Utilisation selon la revendication 1, dans laquelle l'agent nettoyant est un agent d'élimination d'encre.

4. Utilisation selon la revendication 1, dans laquelle l'agent nettoyant est un agent d'élimination de fondant.

5. Utilisation selon la revendication 1, dans laquelle l'agent nettoyant est un agent nettoyant de cristal liquide.

6. Utilisation selon la revendication 1, dans laquelle l'agent nettoyant est un agent décapant de résist.

7. Utilisation selon l'une quelconque des revendications 1 à 6, dans laquelle ladite composition de solvant comprend en plus au moins un composé choisi parmi l'eau, le méthanol, l'éthanol, l'alcool isopropylique, le butanol, le méthylisobutylcarbinol, l'heptanol, l'octanol, le nonanol, le 3-méthylbutanol, le propylèneglycol, le 3-méthoxybutanol, le 3-méthyl-3-méthoxybutanol, le 3,5,5-triméthyl-1-hexanol, le 2-éthyl-1-hexanol, le cyclohexanol, l'alcool benzylique, l'acétone, la méthyléthylcétone, la méthylisobutylcétone, la cyclohexanone, l'isophorone, la pyrrolidone, la N-méthylpyrrolidone, le diméthylformamide, le diméthylacétamide, le diméthylsulfoxyde, le sulfolane, l'acétate de méthyle, l'acétate d'éthyle, l'acétate de butyle, l'acétate d'amyle, l'acétate de 3-méthoxybutyle, l'acétate de 3-méthyl-3-méthoxybutyle, le lactate de méthyle, le lactate d'éthyle, le lactate de butyle, le 3-méthoxypropionate de méthyle, le 3-éthoxypropionate d'éthyle, l'acétate de 2-éthylhexyle, l'acétate de cyclohexyle, l'acétate de benzyle, le dibenzyléther, le nitrométhane, le nitroéthane, l'acétonitrile, la γ-butyrotactone, l'acétate de monométhyléther de propylèneglycol, le toluène, le xylène, l'hexane et le cyclohexane.

8. Utilisation selon l'une quelconque des revendications 1 à 7, dans laquelle ledit ester d'acide oxyisobutyrique est choisi parmi l'a-méthoxyisobutyrate de méthyle, l'α-méthoxylsobutyrate d'éthyle, l'α-éthoxyisobutyrate de méthyle, l'α-éthooyisobutyrate d'éthyle, le β-méthoxyisobutyrate de méthyle, le β-méthoxylsobutyrate d'éthyle, le β-éthoxyisobutyrate de méthyle, le β-éthoxyisobutyrate d'éthyle, le β-isopropoxyisobutyrate de méthyle, le β-isopropoxyisobutyrate d'éthyle, le β-isopropoxyisobutyrate de butyle, le β-butoxylsobutyrate de méthyle, le β-butoxyisobutyrate d'éthyle, le β-butoxyisobutyrate de butyle, l'α-hydroxyisobutyrate de méthyle, l'α-hydroxyisobutyrate d'éthyle, l'α-hydroxyisobutyrate d'isopropyle et l'α-hydroxyisobutyrate de butyle.
